# EUROPEAN PATENT SPECIFICATION

(11) **EP 1 827 136 B1**
(45) Date of publication and mention of the grant of the patent: **04.06.2008**
(21) Application number: 05792107.4
(22) Date of filing: 20.09.2005
(51) Int. Cl.: A23L 1/30

(54) **FORMULATION FOR ORAL ADMINISTRATION HAVING A HEALTH-PROMOTING EFFECT ON THE CARDIOVASCULAR SYSTEM**
FORMULIERUNG FÜR DIE ORALE VERABREICHUNG MIT GESUNDHEITSFÖRDERNDER WIRKUNG AUF DAS HERZ-KREISLAUF-SYSTEM
FORMULATION DESTINEE A ETRE ADMINISTREE PAR VOIE ORALE PRESENTANT UN EFFET BENEFIQUE SUR LE SYSTEME CARDIOVASCULAIRE

(30) Priority: 07.10.2004 IT TO20040682
(43) Date of publication of application: 05.09.2007
(73) Proprietor: ROTTAPHARM S.P.A., 20122 Milano (MI) (IT)
(72) Inventor: SENIN, Paolo, I-20052 MONZA (Milano) (IT); SETNIKAR, Ivo, I-20146 MILANO (IT); ROVATI, Luigi, Angelo, I-20052 MONZA (Milano) (IT)
(74) Representative: Rambelli, Paolo
(86) International application number: PCT/EP2005/054691
(87) International publication number: WO 2006/037725

(56) References cited:
- WO-A-03/020260
- US-A- 5 952 393
- US-B1- 6 541 006

## Description

The present invention relates to a formulation for oral administration, in the form of tablets, capsules and powder for extemporaneous use, supplied in sachets, which can exert a health-promoting effect on the cardiovascular system, owing to a combination of activities, such as eulipidaemic and antioxidant action and protective effects on the vasal endothelium and other effects associated with these directly or indirectly.

Cardiovascular diseases are now among the main causes of disability and mortality in highly-developed countries and many of them are of an arteriosclerotic nature, i.e. due to narrowing of the lumen of the arteries as a result of deposition of cholesterol and other fatty substances present in the blood, of calcium and of fibrin on the vessel walls (atherosclerosis).

Numerous factors are involved in cardiovascular diseases, and are identified as "cardiovascular risk factors" (CVRF).

One of the most important cardiovascular risk factors is blood cholesterol, as has been demonstrated by extensive epidemiological studies which showed a direct correlation between atherosclerosis and total blood cholesterol levels (TC) and, in particular, levels of cholesterol bound to low-density lipoproteins (LDL-C).

Another cardiovascular risk factor is raised by blood levels of homocysteine. Homocysteine is an amino acid that is normally present in the blood, but which - at blood concentrations above 135 mg/dl - is correlated with coronary, cerebral and peripheral vascular diseases and with deep vein thrombosis.

Cardiovascular risk is also increased considerably by the aggressive action of so-called free radicals, i.e. molecules or groups of atoms containing an unpaired electron. This feature makes such substances extremely aggressive with respect to other molecules, from which they remove an electron to restore their original stable configuration, causing severe organic damage such as premature and generalized aging, degenerative diseases of the nervous system, tumours and an increased cardiovascular risk.

Various factors contribute to causing the aforementioned changes in blood composition, and in this sense serious consideration is given to heredity, unbalanced nutrition, with consequent deficit or deficiency of essential nutrients, unsuitable lifestyle and chronic intoxication due to tobacco smoking and other food contaminants and environmental pollutants.

Many risk factors can be reduced or eliminated with correct nutrition, by abstaining from smoking, with regular physical exercise and with a healthy life in the open air.

However, it is not easy to make radical changes to lifestyle and nutrition. On the other hand, for mild or moderate hypercholesterolaemia it is not recommended to initiate therapy with the powerful cholesterol-lowering drugs now available, which can have considerable side-effects.

It may, however, be advantageous to combine the normal diet with natural substances that are able to reduce the cardiovascular risk and supply our body with substances that are missing from normal nutrition which can have a health-promoting effect.

US5952393 relates to a composition for reducing serum cholesterol in humans and animals, including phytosterols and policosanol.

US6541006 provides compositions and methods related to the administration of red yeast rice, coenzyme Q10, chromium and mixed tocopherols to reduce or control blood cholesterol.

The present invention is based on the research and identification of a novel combination of natural active principles, which should exert eulipidaemic and antioxidant effects, counteracting homocysteinaemia, protective action on the vasal endothelium and other effects, supporting and complementing those mentioned; owing to the synergistic action, the aforementioned health-promoting effects are obtained even at relatively low doses, remaining within the specifications for food supplements.

The synergistic composition according to the invention and its use for the preparation of medicinal products and/or compositions of food supplements, having the health-promoting effects mentioned above, are defined in the claims hereunder.

### Policosanols

### Chemistry

"Policosanols" is the generic name assigned to a mixture of saturated long-chain (C₂₂-C₃₆) primary aliphatic alcohols, which are in the form of a solid, of a waxy consistency, sparingly soluble in H₂O. The alcohols of which they are composed occur naturally in the wax produced by bees *(Apis mellifera*), in the waxy matrix of the sugar cane *(Saccharum officinarum),* in rice bran *(Oryza saliva)* and in various other plants.

The policosanols used in the present invention, in contrast to those normally obtained using the conventional wet processes that do not guarantee their purity and the complete removal of the organic solvents employed in the extraction process, are preferably extracted using carbon dioxide in the supercritical state at very low temperature in the liquid phase.

This process guarantees their purity, the original chemical structure, complete absence of potentially contaminating traces of organic solvents and preservation of the original proportions of the alcohols naturally present in the starting product.

### Eulipidaemic effect

Numerous clinical and experimental studies have documented the eulipidaemic action of the policosanols, which is especially apparent in subjects with hypercholesterolaemia. The effect is most pronounced on cholesterol bound to low-density lipoproteins (LDL-C) with inhibition of the synthesis of cholesterol itself and increase in its elimination [Menendez et al. 1994⁽¹⁾, Menendez et al. 1996⁽²⁾, Menendez et al. 2001⁽³⁾].

Investigations into the mechanism of action of the policosanols provide evidence in favour of inhibition of cholesterol biosynthesis in a stage that is between the utilization of the acetate and the production of the mevalonate, though excluding a direct effect on 3-hydroxy-3-methylglutaryl coenzyme A reductase (HMGCoA-r) [Menendez et al. 1996⁽²⁾, Menendez et al. 2001⁽³⁾] which is, however, affected by other well-known cholesterol-lowering drugs, for example the statins.

The policosanols, moreover, produce a significant, dose-dependent increase in the binding and hepatic uptake of LDLs, with a consequent positive acceleration of their catabolism [Menendez et al. 1994⁽¹⁾]. Finally, it has been hypothesized that the policosanols have an action on the intestinal mucosa, relating to the absorption and intracellular metabolism of food lipids [Goumi-Berthold et al. 2002⁽⁴⁾].

### Inhibition of the peroxidation of the LDLs

Various studies in vitro and in vivo have demonstrated that the policosanols exert an antioxidant action with consequent inhibition of peroxidation of LDLs and VLDLs (very low density lipoproteins) [Menendez et al. 1999⁽⁵⁾]. Blocking of peroxidation of the LDLs, which has also been confirmed in human clinical trials [Menendez et al. 2000⁽⁶⁾], leads to an important antiatherogenic effect, because the LDLs only exert their atherogenic action in the oxidized state. This is in fact a necessary structural condition for the LDLs to be able to filter through the endothelium of the arteries to be captured by the receptor for the scavengers of the macrophages.

The latter are then transformed into "foam cells", before entering the formative process of the atheromatous plaques.

### Endothelial protection

A further function of the policosanols is to protect the vasal endothelium from damage caused by mechanical and chemical agents [Benitez et al. 1997⁽²²⁾]. Epithelial damage involves a loss of membrane elasticity and an increase in permeability, which among other things promotes passage of the LDLs from the blood to the arterial intima, with consequent formation of atheromatous plaques.

### Pro-energetic action

Clinical research, conducted since the 1950's, has shown that taking policosanols has beneficial effects on resistance to stress and fatigue. This action relates to the bioavailability of glycogen in muscle, nerve reaction times, resistance to hypoxic stress, oxygen supply and, not least, blood cholesterol [Cureton 1972⁽⁸⁾].

The policosanols are therefore used by athletes and sportspersons in general for their pro-energetic effect, which would be explained by their role as "intracellular vehicle" for the essential fatty acids and for the cell's other energy sources.

### Posology

The daily dose used in the invention is preferably 5-40 mg (usually 10-20), preferably taken with the evening meal for optimum absorption of the fatty alcohols. To be effective, treatment must extend over at least six weeks.

### Red yeast

### Chemistry

Red yeast, or red yeast rice, is the product of fermentation of rice by the yeast *Monascus purpureus,* which produces various substances, including a red pigment (whence the name "red yeast"), bacteriostatic agents and substances that alter the blood lipid level.

These latter are the most interesting components owing to their health-promoting effect. They have been given the name "monacolins". The most effective monacolin for eulipidaemic purposes is monacolin K [Endo 1988⁽⁹⁾] whose content in red yeast depends on the strain of *Monascus purpureus* used and the fermentation conditions.

The red yeast used in the formulation according to the present invention is preferably obtained in standardized fermentation conditions, with a particular strain of *Monascus purpureus,* selected for an optimum yield of monacolin K and a content of said active principle maintained within defined limits.

### Eulipidaemic effect

Blood cholesterol is the resultant of the sum of the intake in food and that arising from endogenous biosynthesis in the liver. This synthesis starts with acetyl coenzyme A, from which there is formation of 3-hydroxy-3-methylglutaryl coenzyme A (HMG-CoA) and then, through the action of HMG-CoA reductase (HMG-CoA-r), the mevalonate. The mevalonate is the key intermediate for the cascade of successive stages of cholesterol biosynthesis and represents the critical and limiting step for the endogenous production and for the relative blood levels of cholesterol itself and of the LDLs.

Monacolin K competes structurally at the level of the HMG-CoA-r with HMG-CoA, the precursor of mevalonate, and has an activity for the enzyme that is 2000 times greater than that of the natural substrate, and therefore activates a mechanism of "competitive enzymatic inhibition" which obstructs the biosynthesis of cholesterol [Man et al. 2002⁽¹⁰⁾].

### Posology

The daily dose of red yeast used in the invention is preferably equivalent to an amount of monacolin between 1 and 6 mg, and preferably does not exceed the equivalent of 3 mg of monacolin in the compositions according to the invention, to be used as food supplement.

### Folic acid (FA)

### Chemistry

FA comprises a combination of three molecules: glutamic acid, p-aminobenzoic acid and a derivative of pteridine.

Our body is not able to synthesize FA and therefore we are dependent on FA-rich food sources such as leafy vegetables, liver, eggs and legumes. The recommended daily requirement of FA in the adult is 0.2 mg and increases to 0.4 mg in pregnancy and to 0.3 mg during lactation.

### Corrective effect on hyperhomocysteinaemia

FA plays an essential role in many biological functions, such as the synthesis of the nucleic acids DNA and RNA, production and development of red blood cells, proper development of the embryo in pregnancy, improvement of trophism and of the state of health of the skin, stimulation of milk secretion, regulation of iron metabolism etc.

Furthermore, FA corrects hyperhomocysteinaemia, a cardiovascular risk factor only slightly less important than hypercholesterolaemia for coronary, cerebral and peripheral vascular diseases and for deep vein thrombosis [Longo 2001⁽¹¹⁾]. The action of FA is connected with its property as a donor of methyl groups which convert homocysteine to L-methionine and S-adenosylmethionine, eliminating the excess homocysteine in the blood.

### Posology

The dosage forms according to the invention are preferably designed for the administration of folic acid at daily doses from 0.1 to 0.6 mg, and more preferably from 0.15 to 0.4 mg.

### Astaxanthin (AX)

### Chemistry

AX is a reddish-orange pigment that is present in many living beings and especially in aquatic animals. Chemically it is a xanthophyll, belonging to the carotenoid family, of formula 3,3'-dihydroxy-β,β'-carotene-4,4'-dione and with two chiral centres on the two cyclic carbons (3 and 3') bound to the hydroxyls.

The hydroxyls are important because they function as points of attachment for other molecules, such as plasma proteins and lipoproteins, and because they endow AX with a bipolar character, so that it can form hydrogen bonds with the polar sites both inside and outside the phospholipid double layer forming the cell membrane, and is thus able to align transversely to said membrane.

AX is thus able to capture and inactivate the free radicals which attack cells from the outside and those that attack them from the inside, efficiently protecting the tissues against damage by free radicals and generally against all agents with an oxidizing action.

Owing to these structural characteristics, AX displays antioxidant activity four times greater than that of lutein, ten times greater than β-carotene and even 100-500 times greater than vitamin E.

The AX used in the formulation according to the present invention is preferably an extract obtained from an alga, *Haematococcus pluvialis,* by a special process using supercritical carbon dioxide which is able to preserve the chemical structure of natural AX and concentrate it to 2.5%, stabilizing it in a complex lipid matrix.

### Antioxidant effect

Owing to its powerful antioxidant activity, AX is able to exert a clear protective action against oxidative stress, a known cause of aging, of degenerative diseases of the nervous system and of tumours. It can also be employed advantageously for countering the irritative processes due to the free radicals triggered by solar and ionizing radiation.

AX is also used for protecting the structures of the retina, in particular for the treatment of macular degeneration. Other potential fields of application, connected with inactivation of free radicals, relate to detoxication from tobacco smoke, diets that are unbalanced through excess of fats, environmental pollutants etc.

### Reduction of cardiovascular risk

Cardiovascular pathology is largely a function of atherosclerotic phenomena and thrombotic events. The formation of atheromatous plaques is closely correlated with the type and amount of blood lipids and in particular LDL-cholesterol (LDL-C).

LDL-C is not dangerous in itself, but becomes so following peroxidation by free radicals. Peroxidation increases its affinity for the arterial walls, with consequent infiltration to the intima and capture by the macrophages, which degenerate to "foam cells", the first event in the process of formation of atheromatous plaques and of arteriosclerosis, a primary factor of cardiovascular risk (CVR) [Steinberg 1997⁽⁷⁾].

It is in fact by intervening in this mechanism that AX, owing to its strong antioxidant activity, opposes peroxidation of LDL-C and its transformation to an atheromatous agent [Iwamoto et al. 2000⁽²¹⁾].

### Safety of use

The NOAEL (no observed adverse effect level) for doses of AX repeated daily in the animal is 1340 mg/kg, corresponding to more than 80 g in man. In man the NOAEL is 6 mg.

### Posology

The dosage forms according to the invention preferably contain astaxanthin at daily doses of 0.1-5 mg, and preferably from 0.4 to 2 mg, preferably taken with a meal, to promote intestinal absorption.

### Coenzyme Q10 (CoQ10)

CoQ10 is a substance of lipid character with a quinone-like structure which, because it is so widespread in nature, including the human body, is also called ubiquinone. The main function of CoQ10 is as a cofactor in the electron-transport chain in the series of redox reactions involved in ATP synthesis.

Because all cellular functions depend on an adequate availability of ATP, CoQ10 is essential for all the tissues and organs of our body. Normally it is synthesized in vivo, but situations may develop in which its endogenous production proves insufficient. In this case deficiency of CoQ10 is felt by all the cells and particularly by those that are metabolically the most active, such as the cardiac cells, those of the nervous system and those of the immune system.

CoQ10 deficiency is therefore directly linked to a whole series of dysfunctions such as cardiovascular diseases, arterial hypertension and AIDS.

### Indications

CoQ10 has proved useful in cardiovascular diseases because it improves the energy output of the myocardium and of the vasal musculature. CoQ10 has also been used with brilliant results in the treatment of cancer [Lockwood et al. 1994⁽¹²⁾, Lockwood et al. 1995⁽¹³⁾] and of muscular dystrophy.

Recently it has also been introduced in sports practice for improving the energy output and the physical performance indices of athletes. Not least in importance is its antioxidant action, with consequent protection of cellular structures against free radicals, with CoQ10 exerting a protective action, synergistically with vitamin E, against damage by oxidative stress.

### Interactions

HMG-CoA-r is necessary for the biosynthesis of CoQ10. Therefore substances that inhibit this enzyme, for example the statins, might at very high doses produce a CoQ10 deficiency state. Thus, investigations in rats and in humans suggest that subjects with low basal levels of CoQ10 and sub-optimum cardiac function can develop excessive CoQ10 depletion after taking HMG-CoA-r inhibitors [Willis RA et al. 1990⁽¹⁴⁾, Folkers K et al. 1990⁽¹⁵⁾].

It is therefore concluded that CoQ10 supplement is always useful in subjects being treated with HMG-CoA-r inhibitors, and especially in subjects with CVR, because CoQ10 improves the energy output of the myocardium and of the vasal musculature.

### Posology

The daily dose of CoQ10, used in the present invention, is preferably in the range 1-20 mg, and preferably from 2 to 4 mg.

### Advantages of the synergistic effects

The formulations according to the invention, in the combinations of active principles contemplated in the accompanying claims, produce beneficial synergistic effects on the following activities.

### Eulipidaemic activity

The active principles contributing to this activity are the policosanols and the red yeast, both of which are involved in the biosynthesis of cholesterol, but with the difference that the former reduce its synthesis from the acetate, acting upstream of 3-hydroxy-3-methylglutaryl coenzyme A reductase (HMG-CoA-r), by interfering with its expression, whereas the latter exert competitive antagonism with the HMG-CoA-r itself, inhibiting the synthesis of the sequence: Acetate-Mevalonate-Cholesterol.

Combining the two active principles produces a synergistic action in blocking the synthesis of cholesterol upstream and downstream of HMG-CoA-r, with the advantage of mutual reinforcement of the eulipidaemic action.

### Endothelium-protecting activity

The vasal endothelium is a delicate biological membrane that permits the passage of substances that diffuse easily, such as respiratory gases, simple carbohydrates and amino acids, but does not allow substances of high molecular weight to pass through, such as proteins, complex lipids and polymeric carbohydrates.

Endothelial damage caused by mechanical stress or aggressive chemical action compromises the characteristics of semipermeability of the endothelium so that molecules of high molecular weight can also pass through. These include the lipoproteins and in particular the LDLs, which after passing through the endothelial filter are captured by the macrophages of the arterial intima, transforming them into foam cells, the initial nucleus of the atherosclerotic plaques.

The compositions according to the invention containing policosanols, folic acid and astaxanthin protect the endothelium, by various mechanisms, against mechanical stress and free radicals, enabling it to maintain its function of semipermeability and preventing the formation of atheromatous plaques and their subsequent development into arteriosclerotic lesions.

### Correction of hyperhomocysteinaemia

The embodiments of the invention that include folic acid in the composition, preferably at the daily doses stated above, prove advantageous in limiting the CVR factor connected with existing or latent hyperhomocysteinaemia.

### Antioxidant activity

The compositions according to the invention, which are particularly useful for the prevention of cardiovascular risk - connected with various kinds of oxidative and/or peroxidative phenomena - contain, in addition to the policosanols, which - aside from the eulipidaemic action already mentioned - also exert an effective antioxidant effect, also astaxanthin and coenzyme Q10 which in their turn are able to exert an antioxidant and protective activity on the cellular structures, against potential damage caused by attack by free radicals.

### Synergistic action of CoQ10:

CoQ10 is essential for the energy efficiency of the cellular functions and its deficiency is felt in particular by subjects with reduced or compromised cardiac function and by subjects using HMG-CoA-r inhibitors. CoQ10 is preferably added to the formulation in amounts such as to ensure a perfectly safe tissue level and to optimize the energy output of the heart and of the vasal musculature.

### EXPERIMENTAL SECTION

The inventive aim of the present patent application was verified by evaluating and comparing, on an experimental model of atherosclerosis, the individual and combined effect of some of the more important active principles described above, according to the following experimental plan.

### Materials and methods

### Principle of the method

The effect of the substances under investigation, enumerated earlier, was evaluated by administration to rabbits made hypercholesterolaemic with a diet with added cholesterol according to Prasad et al. [1993⁽¹⁶⁾].

### Animal experiments

The doses used for policosanols, red yeast and astaxanthin for the treatment of hypercholesterolaemia induced in the rabbit described in the experimental section are about four times greater than those used and described in the section devoted to the examples of formulation according to the present invention.

The difference in posology between animals and humans is perfectly normal in pharmacology, because it is well known that pathologies induced experimentally in animals are always dramatically and unnaturally more severe than those that develop spontaneously in humans.
- Animals: New Zealand White rabbits of 6-8 weeks and with body weight from 2 to 3 kg.
- Feeding:
   - *Normal diet:* standard diet for guinea-pigs and rabbits according to Sherman, in pellets (Laboratorio dottori Piccioni S.n.c.).
   - *Atherogenic diet:* standard diet for guinea-pigs and rabbits according to Sherman, with 7 g/kg of cholesterol mixed in, in pellets.
      The diets were rationed at the rate of 150 g of feed per day. Water was supplied *ad libitum.*
- Substances: the test substances were administered with a stomach tube, once a day, suspended in 2 ml/kg of carboxymethylcellulose 0.2% (CMC).
   - *Policosanols (P):* Policosanols is the generic name given to a mixture of long-chain saturated primary aliphatic alcohols.
   - *Red yeast (E):* A standardized extract containing 1.5% of monacolin K was used.
   - *Astaxanthin (A):* A concentrate of cultures of *Haematococcus pluvialis* (EHP) containing 2.5% of astaxanthin was used.
- Treatments and groups: All the animals were acclimatized in the animal breeding department at 25±2°C and humidity of 60%-70%. After one week, blood samples were taken for the baseline data. Next, the animals were randomized into the following treatment groups, each of ten rabbits.
   1. (C) Controls on normal diet: daily administration of 2 ml/kg of CMC by stomach tube.
   2. (AT) Animals fed with atherogenic diet: daily administration of 2 ml/kg of CMC by stomach tube.
   3. (P) Atherogenic diet with policosanols: daily administration, by stomach tube, of 0.6 mg/kg of policosanols, suspended in 2 ml/kg of CMC.
   4. (E) Atherogenic diet with red yeast: daily administration, by stomach tube, of 12 mg/kg of red yeast (0.18 mg/kg of monacolin equivalents), suspended in 2 ml/kg of CMC.
   5. (P+E) Atherogenic diet with policosanols and red yeast: daily administration, by stomach tube, of 0.6 mg/kg of policosanols + 12 mg/kg of red yeast, suspended in 2 ml/kg of CMC.
   6. (P+E+A) Atherogenic diet with policosanols, red yeast and astaxanthin: daily administration, by stomach tube, of 0.6 mg/kg of policosanols + 12 mg/kg red yeast + 1.2 mg/kg of EHP (0.03 mg/kg of astaxanthin), suspended in 2 ml/kg of CMC.
   The treatments were continued for three months. Blood samples for the analyses were taken after one, two and three months. At the end of the third month, the animals were sacrificed for the morphological observations.
- Blood analysis:
   - Total cholesterol in the serum (TC)
   - Cholesterol bound to HDLs (HDL-C)
   - Cholesterol bound to LDLs (LDL-C)
   - Triglycerides
      These analyses were carried out by the usual laboratory methods.
   - Blood malondialdehyde (MDA)
      MDA is an index of the degree of peroxidation of the lipids and increases in the blood in the presence of atherosclerosis and hypercholesterolaemia [Prasad et al. 1989⁽¹⁷⁾]. MDA was assayed as "tiobarbituric acid-reactive substance" according to Prasad et al. [1990⁽¹⁸⁾].
- Morphological examination of the aorta: The aorta was removed from its origin as far as the iliac bifurcation, opened lengthwise and prepared for examination of lipid deposits on the intima by staining with Sudan IV according to Holman et al. [1958⁽¹⁹⁾]. The sudanophilic (atherosclerotic) area, expressed as a percentage of the total area of the aorta, is used as the evaluation parameter.
- Statistical analysis: The mean values and the standard errors of the mean values were calculated for the variables examined. The Student test was used for evaluating the significance of the comparisons.

### Results

### Body weight

During the three months of the study, a significant increase in body weight occurred in all the groups. The increase was 51% in the controls, 76% in the animals with atherogenic diet, 54% in those with atherogenic diet and administration of policosanols, 44% in those with atherogenic diet and administration of red yeast, 52% in those with atherogenic diet and administration of policosanols and red yeast, and 63% in those with atherogenic diet and administration of policosanols, red yeast and astaxanthin.

The difference in growth relative to the controls did not reach statistically significant values in any group.

### Effects on blood cholesterol

The atherogenic, hypercholesterol diet (Group 2) caused a very large increase in TC, above all through an increase in LDL-C, so that the ratio HDL/TC x 100 decreased from the baseline value of 63, to 14 (Pₜ < 0.001).

Treatment with policosanols (Group 3 - 0.6 mg/kg/d of policosanols) reduced the increase in TC by 5 mmol/l, but this is not statistically significant. However, the 9 mmol/l decrease in LDL-C is significant (Pₜ < 0.05). The HDL/TCx100 ratio also improved, increasing from the value of 14, observed in Group 2, to 21. The difference, however, did not reach statistically significant levels.

Treatment with red yeast (Group 4 - 0.18 mg/kg/d of monacolin equivalents) reduced the increase in TC by 8 mmol/l, but again this was not statistically significant. However, the decrease in LDL-C, of 13 mmol/l, was significant (Pₜ < 0.005) and greater than that observed with policosanols. The HDL/TCx100 ratio also improved, increasing from the value of 14 observed in Group 2, to 25. However, the difference did not reach statistically significant levels.

Treatment with policosanols combined with red yeast (Group 5) reduced the increase in TC significantly (Pₜ < 0.001), by 21 mmol/1. The decrease in LDL cholesterol, of 28 mmol/1, was greater than the sum of that with policosanols and with red yeast, demonstrating potentiation of the effect of the components taken individually. The HDL/TCx100 ratio also improved considerably, increasing from a value of 14, observed in Group 2, to 34, with a significance of Pₜ < 0.001.

The combination with astaxanthin (Group 6 - with 0.03 mg/kg/d of astaxanthin, added to the policosanols and to the red yeast) was even more effective. This treatment reduced the increase in TC significantly (Pₜ < 0.001), by 21 mmol/dl. The decrease in LDL-C, of 30 mmol/1, was slightly greater than that observed in Group 5, again showing potentiation of the effect of the policosanols and of the red yeast.

The decrease was very significant in this group too (Pₜ < 0.001). There was particular improvement in the HDL/TCx100 ratio, rising from the value of 1 observed in Group 2, to 39, with a significance of Pₜ < 0.001.

### Effects on triglycerides

The atherogenic diet led to an increase in triglycerides relative to the controls of 1.58 mmol/l (136%). This is a considerable increase, but it is not statistically significant, owing to the high variability of this parameter in the blood. The policosanols and the other substances administered decreased, but not significantly, the increase in triglycerides produced by the atherogenic diet.

### Effects on blood malondialdehyde (MDA)

The atherogenic diet caused an increase of 2.01 mmol/l (87%) in blood MDA relative to the controls. The increase is significant (P < 0.001), showing that the atherogenic diet has oxidizing effects on the blood lipids.

The policosanols reduced the increase in MDA by 0.35 mmol/l (11%). This decrease is not, however, statistically significant.

The red yeast reduced the increase in MDA induced by the atherogenic diet by 0.71 mmol/l (23%), which is a statistically significant decrease (P < 0.01).

The combination of policosanols with red yeast reduced the increase in MDA induced by the atherogenic diet by 0.95 mmol/l (31%). The decrease is statistically significant (P < 0.001) and corresponds to the sum of the antioxidant effect produced by the policosanols and the red yeast.

Addition of astaxanthin to the aforesaid combination reduced the increase in MDA induced by the atherogenic diet by 1.51 mmol/l (49%). This decrease, which is statistically very significant (P < 0.001), represents potentiation of the antioxidant effect of the policosanols and the red yeast.

### Effects on sudanophilic infiltrations in the aorta

The atherogenic diet with cholesterol caused sudanophilic infiltration affecting 74±8 percent (mean value ± SE) of the wall of the aorta. In contrast, infiltration was practically absent in the controls.

The policosanols reduced the sudanophilic infiltration from atherogenic diet by 11% and this decrease is not statistically significant.

The red yeast, in its turn, reduced the sudanophilic infiltration from atherogenic diet by 20% but once again the decrease is not statistically significant.

The combination of policosanols with red yeast reduced the sudanophilic infiltration from atherogenic diet by 33%, showing summation of the effects of the two active principles. This effect was statistically significant in this case (P < 0.05).

Addition of astaxanthin to the policosanols-red yeast combination induced a significant (P < 0.001) further potentiation of the anti-atherogenic effect, manifested by a 92% decrease in sudanophilic infiltration from atherogenic diet.

These results show that the eulipidaemic action of the policosanols and of red yeast reduces the sudanophilic infiltration from atherogenic diet and that their effects are greatly enhanced by the strong antioxidant property of astaxanthin.

### Discussion

### Effects of the treatments on total cholesterol (TC), on LDL cholesterol (LDL-C) and on HDL cholesterol (HDL-C)

In the model of atherosclerosis used in this study, based on administration of cholesterol-enriched feed for three months, blood TC rose from 1.5 to 69.8 mmol/l, LDL-C from 0.6 to 60 mmol/l and HDL-C from 1.0 to 9.8 mmol/l. The latter, however, increased much less than TC, for which the index of anti-atherogenic protection represented by the ratio HDL/TC x 100 fell from 67 to 14.

The TC that had been increased by the atherogenic diet was lowered by 7% by policosanol, at daily dose of 0.6 mg/kg, and by 11% by red yeast, containing a daily dose of 0.18 mg/kg of monacolin. The policosanols - red yeast combination lowered the TC, which had been increased by the atherogenic diet, by 30%, showing a surprising potentiation of the cholesterol-lowering effects produced by the two active principles taken individually.

This potentiation can be explained *a posteriori,* if we take into account the respective mechanisms of action: in fact, the policosanols interfere with expression and degradation of 3-hydroxy-3-methylglutaryl coenzyme A reductase (HMG-CoA-r) [Menendez et al. 2001⁽³⁾], whereas monacolin exerts competitive antagonism with respect to HMG-CoA-r [Herber et al. 1999⁽²⁰⁾].

The two mechanisms are clearly independent of one another and potentiate each other (HMG-CoA-r, as is well known, is the enzyme necessary for the synthesis of mevalonate, the precursor of cholesterol). Similar considerations apply to LDL-C, which is also lowered in parallel with TC by policosanol, by red yeast and by the policosanols-red yeast combination.

HDL-C, relative to the values observed after the atherogenic diet, was increased, not statistically significantly, by 41% and 56% through the action of the policosanols and red yeast, respectively, with an increase in the ratio HDL/TC x 100, the index of anti-atherogenic protection, of 52% and 76%.

The combination of policosanols with red yeast produced a 70% increase in HDL-C and a 144% increase in the ratio HDL/TC x 100, thus showing a significant improvement in anti-atherogenic protection relative to that of the individual active principles. Addition of a daily dose of 0.03 mg/kg of astaxanthin to the aforesaid combination caused a significant increase of 91% in HDL-C and of 174% in the ratio HDL/TC x 100.

The combination of red yeast with policosanols thus proves very effective in controlling the various atherogenic parameters connected with blood cholesterols and addition of astaxanthin produces a further improvement in their anti-atherogenic efficacy.

### Effects of the treatments on triglycerides

Feeding rabbits for three months with cholesterol-enriched feed caused an increase in triglycerides from 0.9 to 2.2 mmol/l. The increase was not statistically significant, however, owing to the high variability of this parameter. The model does not seem suitable for investigating possible effects on triglycerides. The various treatments reduced the increase in triglycerides caused by the atherogenic feed. The decreases were not found to be statistically significant, owing to the unsuitability of the experimental model.

### Effects of the treatments on blood malondialdehyde (MDA)

MDA is the product of peroxidation of blood lipids and is therefore an important indicator of peroxidation of LDLs by free radicals, a chemical reaction that makes LDLs atherogenic [Prasad et al. 1989⁽¹⁷⁾].

Feeding rabbits for three months with cholesterol-enriched feed caused MDA to increase from 0.99 to 3.11 mmol/l, confirming the atherogenic capacity of this diet with respect to this parameter as well.

MDA, which had increased under the action of the atherogenic feed, was lowered by 11% by the policosanols and by 23% by the red yeast. The combination of the two active principles lowered MDA by 31%, thus showing summation of the effects. MDA, increased by the atherogenic feed, was lowered to 49% by astaxanthin, added to the aforesaid combination, thus showing an exceptional capacity for preventing the peroxidation of blood lipids and hence also of the atherogenic potential of the cholesterol-enriched diet.

### Effects of the treatments on sudanophilic lipid infiltration (SLI) in the intima of the aorta

Feeding of rabbits with cholesterol-enriched feed for three months caused SLI to increase until 74% of the area of the affected aorta.

SLI, increased by the effect of cholesterol-enriched feed, was reduced by 12% and 20% by policosanols and by red yeast, respectively. The combination of the two active principles produced a statistically significant (P < 0.05) 33% decrease in SLI, showing summation of the effects of the two active principles taken individually. The efficacy of addition of astaxanthin to the combination was very pronounced, causing a 92% decrease in SLI that had been increased by the effect of cholesterol-rich feed.

SLI is probably the parameter with highest correlation with the atherogenic effect of cholesterol-rich feed, and with the anti-atherogenic effects of the various treatments. It is important to point out the anti-atherogenic effects of the policosanols and of red yeast, as well as the summation of their effects when administered in combination. However, the potentiating effect of addition of astaxanthin is especially important, as the resultant combination almost completely prevents the atherogenicity induced in rabbits by the experimental model used for the evaluation.

### Conclusions

The experimental model of atherosclerosis adopted in this study comprises feeding rabbits, aged six-eight weeks, for three months with feed enriched with 7 g/kg of cholesterol. This feed causes a marked, and very significant, increase in total blood cholesterol (TC), LDL cholesterol (LDL-C) and, to a lesser extent, HDL cholesterol (HDL-C). This leads to a decrease in anti-atherogenic protection, expressed by the ratio HDL/TC x 100.

The policosanols and red yeast reduce the increase in TC and LDL-C, and improve the ratio HDL/TC x 100. Combination of the two active principles potentiates these effects, providing experimental confirmation of the effectiveness of combining them.

The atherogenic diet also increases blood malondialdehyde (MDA), produced by peroxidation of blood lipids, and so is an important indicator of the chemical reaction that makes the LDLs atherogenic. The policosanols and red yeast reduce the increase in MDA and their combination leads to summation of these effects.

Addition of astaxanthin to the aforesaid combination causes further, significant potentiation of the decrease in MDA, revealing the effectiveness of the composition based on policosanols, red yeast and astaxanthin for the purposes of inhibiting the peroxidation of blood lipids and hence their atherogenic capacity.

The capacity of the atherogenic diet to cause sudanophilic lipid infiltration (SLI) in the intima of the aorta should be noted in particular, because this phenomenon is representative, from various standpoints, of the formation of the plaques of human arteriosclerosis. The policosanols and red yeast decrease SLI, their combination produces a summation of this effect and the addition of astaxanthin to the aforesaid combination causes significant potentiation of the decrease in SLI, which restores the aorta to a practically normal situation.

It can therefore be concluded that the combination of natural active principles, represented by the policosanols, red yeast and astaxanthin, exerts a very pronounced anti-atherogenic effect on the experimental model of atherosclerosis employed, owing to summation and potentiation of the respective cholesterol-lowering actions and inhibition of peroxidation of blood lipids.

These results are undoubtedly also applicable in the case of human arteriosclerosis, both on account of the experimental model which reproduces its initial mechanism of pathogenesis, and because the doses of the various active principles, referred to body weight, can be adjusted to doses suitable for human use, bearing in mind that the pathologies induced experimentally in animals are always dramatically more severe than those that are manifested spontaneously in humans.

### Examples

The formulations stated hereunder illustrate possible practical applications of the present invention and as such should not be regarded in any way as limiting said invention.

Attention is drawn in particular to the excipients of the various forms considered, which can be used as alternatives to those explicitly mentioned in the present invention, depending on the requirements of formulation technology, and which can moreover be selected from a very extensive range of commercially available products that are well known to a person skilled in the art of pharmaceutics and therefore require no inventive step.

### Example 1: Tablets for oral use

The formulation shown below relates to active principles and excipients (with description of the corresponding technological function) which can be used in the preparation of tablets for use for oral administration according to the present invention.

The daily dose of the active principles is incorporated in a single tablet whose preparation only requires ordinary operations that are familiar to a person skilled in the art, i.e.:
- Weighing the individual components of the formulation
- Preparation of the compression mixture for dry homogenization in a suitable mixer
- Compression of the resulting mixture in a suitable automatic tabletting machine, finally obtaining tablets of the appropriate shape and dimensions.

| **Active principles** | | **Quantity/tablet** |
|---|---|---|
| Red yeast (*) | : | 200 mg |
| (*Corresponding to Monacolin K*) | : | *(3 mg)* |
| Extract of microalgae (**) | : | 20 mg |
| *(Haematococcus purpureus, corresponding to Astaxanthin)* | : | *(0.5 mg)* |
| Policosanols | : | 10 mg |
| Coenzyme Q10 | : | 2 mg |
| Folic acid | : | 0.2 mg |

| | | |
|---|---|---|
| (*) Rice fermented with *Monascus purpureus* containing 1.5% of Monacolin K. (**) Contains 2.5% of Astaxanthin | | |

| **Excipients** | **Quantity/ tablet** | **Function** |
|---|---|---|
| Dibasic calcium phosphate | 305.8 mg | Glidant/Diluent |
| Microcrystalline cellulose | 242.0 mg | Diluent/ Disintegrant |
| Magnesium stearate | 8.0 mg | Lubricant |
| Mono- and diglycerides of fatty acids | 8.0 mg | Plasticizer |
| Silicon dioxide | 4.0 mg | Adsorbent/ Antiadherent |

### Example 2: Capsules for oral use

The formulation shown below relates to active principles and excipients (with description of the corresponding technological function) which can be used in the preparation of capsules for use for oral administration according to the present invention.

The daily dose of the active principles is incorporated in a single capsule whose preparation only requires ordinary operations that are familiar to a person skilled in the art, i.e.:
- Weighing the individual components of the formulation
- Preparation of the encapsulation mixture for dry homogenization in a suitable mixer
- Final filling, in an automatic capsule filling machine, in hard-gelatin capsules of suitable dimensions and of the desired colour.

| **Active principles** | | **Quantity/capsule** |
|---|---|---|
| Red yeast (*) | : | 200 mg |
| *(Corresponding to Monacolin K)* | : | *(3 mg)* |
| Extract of microalgae (**) | : | 20 mg |
| *(Haematococcus purpureus, corresponding to Astaxanthin)* | : | *(0.5 mg)* |
| Policosanols | : | 10 mg |
| Coenzyme Q10 | : | 2 mg |
| Folic acid | : | 0.2 mg |

| | | |
|---|---|---|
| (*) Rice fermented with *Monascus purpureus* containing 1.5% of Monacolin K (**) Contains 2.5% of Astaxanthin | | |

| **Excipients** | **Quantity/capsule** | **Function** |
|---|---|---|
| Starch | 60.0 mg | Diluent/ Disintegrant |
| Lactose | 29.0 mg | Diluent |
| Magnesium stearate | 5.0 mg | Lubricant |
| Talc | 2.6 mg | Glidant |

### Example 3: Heat-sealed sachets containing powder for extemporaneous suspension

The formulation shown below relates to active principles and excipients (with description of the corresponding technological function) which can be used in the formulation of a powder for the preparation of extemporaneous suspensions that are to be taken by the oral route. The daily dose is incorporated in a single portion of powder contained in a heat-sealed sachet and comprising an outer layer of paper, an aluminium interface and an inner layer of polyethylene.

The operations of preparation of the pharmaceutical form in question, which are familiar to and applied by any technical personnel in this branch of industry, are essentially as follows:
- Weighing of the individual components of the formulation
- Preparation of the mixture of the components for dry homogenization in a suitable mixer
- Heat-forming of the sachets on a suitable automatic line
- Filling and sealing of the sachets on the same automatic line described above, finally obtaining sachets having the desired shape and dimensions and containing the powder monodose for extemporaneous use.

| **Active principles** | | **Quantity/sachet** |
|---|---|---|
| Red yeast (*) | : | 200 mg |
| *(Corresponding to Monacolin K)* | : | *(3 mg)* |
| Extract of microalgae (**) | : | 20 mg |
| *(Haematococcus purpureus corresponding to Astaxanthin)* | : | *(0.5 mg)* |
| Policosanols | : | 10 mg |
| Coenzyme Q10 | : | 2 mg |
| Folic acid | : | 0.2 mg |

| | | |
|---|---|---|
| (*) Rice fermented with *Monascus purpureus* containing 1.5% of Monacolin K. (**) Contains 2.5% of Astaxanthin | | |

| **Excipients** | **Quantity/sachet** | **Funcion** |
|---|---|---|
| Sorbitol | 815.0 mg | Diluent/Sweetener |
| Citric Acid | 10.0 mg | Enhancement of taste |
| Polyethylene Glycol 4000 | 4.0 mg | Lubricant/Plasticizer |
| Others (*) | q.s.f. | Sweeteners/ Flavourings |

| | | |
|---|---|---|
| (*) Flavourings and sweeteners can be added freely, depending on preferences. | | |

### REFERENCES

[1] Menendez R, Fernandez SI, Del Rio A et al.: Policosanol inhibits cholesterol biosynthesis and enhances low density lipoprotein processing in cultured human fibroblasts. Biol Res 1994; 27:199-203
[2] Menendez R, Amor MR, Gonzales RM et al.: Effects of Policosanol on the hepatic cholesterol biosynthesis of normocholesterolemic rats. Biol Res 1996; 29:253-257
[3] Menendez R, Amor AM, Rodeiro I et al.: Policosanol modulates HMG - CoA reductase activity in cultured fibroblasts. Arch Med Res 2001; 32:8-12
[4] Goumi-Berthold I, Goumi-Berthold H: Policosanol: clinical pharmacology and therapeutic significance of a new lipid - lowering agent. Am Heart J 2002; 143:356-365
[5] Menendez R, Fraga V, Amor AM et al.: Oral administration of Policosanol inhibits in vitro copper ion-induced rat lipoprotein peroxidation. Physiol Behav 1999; 67:1-7
[6] Menendez R, Mas R, Amor AM et al.: Effects of Policosanol treatment on the susceptibility of low density lipoprotein (LDL) isolated from healthy volunteers to oxidative modification in vitro. Br J Clin Pharmacol. 2000; 50:255-262
[7] Steinberg D: Low density lipoprotein oxidation and its pathobiological significance. J Biol Chem 1997; 272; 20963-20966
[8] Cureton K: The physiological effects of wheat germ oil in humans. 1972; In Exercise, Charles C Thomas, Springfield, IL-USA: 296-300
[9] Endo A: Chemistry, Biochemistry and Pharmacology of HMG-CoA reductase inhibitors. Klin Wochensr. 1988; 421-427
[10] Man RY, Lynn EG, Cheung F, Tsang PS.O.K.: Cholestin inhibits Cholesterol synthesis and secretion in hepatic cells (HepG2). Mol Cell Biochem. 2002; 233:153-158
[11] Longo N. In Harrison's 15th Edition. Mc Graw - Hill, New York 2001, p.2306
[12] Lockwood K, Moesgaard S, Hanioka T, Folkers K.: Apparent partial remission of breast cancer in 'high risk' patients supplemented with nutritional antioxidants, essential fatty acids and coenzyme Q10. Molec Aspects Med 1994; 15:S231-240
[13] Lockwood K, Moesgaard S, Yamamoto T, Folkers K.: Progress on therapy of breast cancer with vitamin Q10 and the regression of metastases. Biochem Biophys Res Commun 1995; 212:172-177
[14] Willis RA, Folkers K, Tucker JL et al.: Lovastatin decreases coenzyme Q10 levels in rats. Proc Natl Acad Sci 1990; 87:8928-8930
[15] Folkers K, Langsjoen P, Willis R et al.: Lovastatin decreases coenzyme Q10 levels in humans. Proc Natl Acad Sci 1990; 87:8931-8934
[16] Prasad K, Kalra J.: Oxygen free radicals and hypocholesterolemic atherosclerosis: Effect of Vitamin E. Am. Heart J 1993; 125:958-973
[17] Prasad K, Kalra J.: Experimental atherosclerosis and oxygen free radicals. Angiology 1989; 40:835-843
[18] Prasad K, Kalra J, Chandkhary AK, Debuath D: Effect of polymorphonuclear leukocyte derived oxygen free radicals and hypochlorous acid on cardiac function and some biochemical parameters. Am.Heart J 1990; 119:538-550
[19] Holman RS, Mc Gill HC jr., Strong JP, Greer JC: Technics for studying atherosclerotic lesions. Lab Invest 1958; 7:42-47
[20] Herber D, Yip I, Ashley JM, Elashoff DA, GoVLW.: Cholesterol-lowering effects of a proprietary Chinese red-yeast-rice dietary supplement. Am J Clin Nutr 1999; 69:231-236
[21] Iwamoto T, Hosoda K, Hirano R, Kurata H, Matsumoto A, Miki W, Kamiyama M, Itakura H, Yamamoto S, Kondo K: Inhibition of low density lipoprotein oxidation by Astaxanthin. J Atheroscler Thromb 2000; 7:216-222
[22] Benitez M, Romero C, Mas R et al.: A comparative study of policosanol versus pravastatin in patients with type II hypercholesterolemia. Curr Ther Res Clin Exp 1997; 58:859-867.

## Claims

1. Composition for oral administration having a health-promoting effect on the cardiovascular system, **characterized in that** it comprises, as active principles, a policosanol or a mixture of policosanols, red yeast and an agent selected from astaxanthin and folic acid.

2. Composition according to Claim 1, further comprising coenzyme Q10.

3. Composition according to Claim 1 or 2, **characterized in that** it comprises, as source of astaxanthin, an extract of microalgae, particularly of *Haematococcus purpureus.*

4. Composition according to any one of the Claims 1 to 3, in the form of dosage units suitable for the administration of policosanols at daily dose from 5 to 40 mg, preferably from 10 to 20 mg.

5. Composition according to any one of the Claims 1 to 3, in the form of dosage units suitable for the administration of red yeast at daily dose equivalent to from 1 to 6 mg of monacolin, preferably not greater than an equivalent of 3 mg of monacolin.

6. Composition according to any one of the Claims 1 to 5, in the form of dosage units suitable for the administration of astaxanthin at daily dose from 0.1 to 5 mg, preferably from 0.4 to 2 mg.

7. Composition according to any one of the Claims 1 to 6, in the form of dosage units suitable for the administration of folic acid at daily dose from 0.1 to 0.6 mg, and preferably from 0.15 to 0.4 mg.

8. Composition according to any one of the Claims 1 to 7, in the form of dosage units containing coenzyme Q10 and suitable for the administration of coenzyme Q10 at daily doses from 1 to 20 mg, and preferably 2 to 4 mg.

9. Composition according to any one of the preceding claims, in the form of tablets, capsules or powder for extemporaneous suspension.

10. Use of a composition according to any one of the Claims 1 to 9, for the preparation of a medicinal product or of a food supplement having eulipidaemic activity, correction of homocysteinaemia, antioxidant action and protective effect on the vasal endothelium.

## Patentansprüche

1. Zusammensetzung zur oralen Verabreichung mit einer gesundheitsfördernden Wirkung auf das Herz-KreislaufSystem, **dadurch gekennzeichnet, dass** die Zusammensetzung als aktive Grundbestandteile ein Policosanol oder ein Gemisch aus Policosanolen, rote Hefe (red yeast) sowie ein Agens enthält, das aus Astaxanthin und Folsäure ausgewählt wird.

2. Zusammensetzung gemäß Anspruch 1, wobei die Zusammensetzung weiters Coenzym Q10 enthält.

3. Zusammensetzung gemäß Anspruch 1 oder 2, **dadurch gekennzeichnet, dass** die Zusammensetzung als Ausgangsstoff des Astaxanthins ein Extrakt aus Mikroalgen enthält, im Besonderen aus *Haematococcus purpureus.*

4. Zusammensetzung gemäß irgendeinem der Ansprüche 1 bis 3 in Form von Dosierungseinheiten, die für die Verabreichung von Policosanolen in einer täglichen Dosis von 5 bis 40 mg geeignet sind, vorzugsweise von 10 bis 20 mg.

5. Zusammensetzung gemäß irgendeinem der Ansprüche 1 bis 3 in Form von Dosierungseinheiten, die für die Verabreichung von roter Hefe (red yeast) in einer täglichen Dosis geeignet sind, die 1 bis 6 mg Monacolin äquivalent ist, vorzugsweise nicht mehr als einem Äquivalent von 3 mg Monacolin.

6. Zusammensetzung gemäß irgendeinem der Ansprüche 1 bis 5 in Form von Dosierungseinheiten, die für die Verabreichung von Astaxanthin in einer täglichen Dosis von 0,1 bis 5 mg geeignet sind, vorzugsweise von 0,4 bis 2 mg.

7. Zusammensetzung gemäß irgendeinem der Ansprüche 1 bis 6 in Form von Dosierungseinheiten, die für die Verabreichung von Folsäure in einer täglichen Dosis von 0,1 bis 0,6 mg geeignet sind, vorzugsweise von 0,15 bis 0,4 mg.

8. Zusammensetzung gemäß irgendeinem der Ansprüche 1 bis 7, in Form von Dosierungseinheiten, die Coenzym Q10 enthalten und für die Verabreichung von Coenzym Q10 in einer täglichen Dosis von 1 bis 20 mg geeignet sind, vorzugsweise von 2 bis 4 mg.

9. Zusammensetzung gemäß irgendeinem der bisherigen Ansprüche in Form von Tabletten, Kapseln oder Pulvern für eine nicht vorbereitete Suspension.

10. Verwendung einer Zusammensetzung gemäß irgendeinem der Ansprüche 1 bis 9 für die Herstellung eines medizinischen Produkts oder eines Nahrungsergänzungsmittels mit einer normalisierenden Wirkung auf den Lipidspiegel, mit einer Korrektur von Homocysteinämie, mit einer antioxidierenden Wirkung sowie mit einer Schutzwirkung auf das Gefäßendothel.

## Revendications

1. Composition pour administration orale présentant un effet bénéfique sur le système cardiovasculaire, **caractérisée en ce qu'**elle comprend, en tant que principes actifs, un policosanol ou un mélange de policosanols, de la levure rouge et un agent choisi parmi l'astaxanthine et l'acide folique.

2. Composition selon la revendication 1, comprenant en outre de la coenzyme Q10.

3. Composition selon la revendication 1 ou 2, **caractérisée en ce qu'**elle comprend, en tant que source d'astaxanthine, un extrait de microalgues, en particulier de *Haematococcus purpureus.*

4. Composition selon l'une quelconque des revendications 1 à 3, sous la forme d'unités posologiques convenables pour l'administration de policosanols à une dose journalière de 5 à 40 mg, de préférence de 10 à 20 mg.

5. Composition selon l'une quelconque des revendications 1 à 3, sous la forme d'unités posologiques convenables pour l'administration de levure rouge à une dose journalière équivalente à une quantité de 1 à 6 mg de monacoline, de préférence pas supérieure à un équivalent de 3 mg de monacoline.

6. Composition selon l'une quelconque des revendications 1 à 5, sous la forme d'unités posologiques convenables pour l'administration d'astaxanthine à une dose journalière de 0,1 à 5 mg, de préférence de 0,4 à 2 mg.

7. Composition selon l'une quelconque des revendications 1 à 6, sous la forme d'unités posologiques convenables pour l'administration d'acide folique à une dose journalière de 0,1 à 0,6 mg, et de préférence de 0,15 à 0,4 mg.

8. Composition selon l'une quelconque des revendications 1 à 7, sous la forme d'unités posologiques contenant de la coenzyme Q10 et convenables pour l'administration de coenzyme Q10 à des doses journalières de 1 à 20 mg, et de préférence de 2 à 4 mg.

9. Composition selon l'une quelconque des revendications précédentes, sous la forme de comprimés, de capsules ou de poudre pour suspension extemporanée.

10. Utilisation d'une composition selon l'une quelconque des revendications 1 à 9, pour la préparation d'un produit médicinal ou d'un complément alimentaire présentant une activité eulipidémique, une correction de l'homocystéinémie, une action antioxydante et un effet protecteur sur l'endothélium vasculaire.
